Europäisches Patentamt

(19) European Patent Office

Office européen des brevets

(11) Publication number: **0 029 349**
**B1**

(12) **EUROPEAN PATENT SPECIFICATION**

(45) Date of publication of patent specification: **05.06.85**

(21) Application number: **80304068.2**

(22) Date of filing: **13.11.80**

(51) Int. Cl.⁴: **A 61 B 5/02**

(54) **Apparatus for automatically measuring blood pressure.**

(30) Priority: **14.11.79 US 94020**

(43) Date of publication of application:
**27.05.81 Bulletin 81/21**

(45) Publication of the grant of the patent:
**05.06.85 Bulletin 85/23**

(84) Designated Contracting States:
**BE CH DE FR GB IT LI NL SE**

(56) References cited:
**DE-A-2 811 362**
**US-A-3 101 082**
**US-A-3 236 230**
**US-A-4 116 230**

**MEDICAL AND BIOLOGICAL ENGINEERING,
vol. 7, nr. 2, March 1969, STEVENAGE (GB), E.
VAN VOLLENHOVEN et al., "Frequency
analysis of heart murmurs", pages 227-231**

(73) Proprietor: **Critikon, Inc.**
**501 George Street**
**New Brunswick, New Jersey (US)**

(72) Inventor: **Maynard, Ramsey III**
**2416 Watrous Avenue**
**Tampa Florida (US)**

(74) Representative: **Colgan, Stephen James et al**
**CARPMAELS & RANSFORD 43 Bloomsbury
Square**
**London WC1A 2RA. (GB)**

Courier Press, Leamington Spa, England.

## Description

This invention relates to an apparatus according to the first part of claim 1. Such an apparatus is known from DE—A—2 811 362.

Background and Prior Art

The most conventional and time honoured method for obtaining blood pressure is the manual ausculatory method, wherein a cuff is inflated to arrest blood flow in a limb, after which the pressure is slowly decreased. During the time of pressure decrease, the clinician, such as by means of a stethoscope, listens to sounds occurring in the limb at a point distal to the cuff, for example at the brachial artery. Re-establishment of circulation is accompanied by pulsating sounds, known as Korotkoff sounds, and the cuff pressures substantially at the beginning and the end of occurrence of these Korotkoff sounds are noted by the clinician as systolic and diastolic blood pressures, respectively.

More recently, methods and apparatus have been proposed whereby blood pressure is evaluated substantially automatically, and, as desired, on a cyclically repeatable basis. Some of these methods involve the application of disparate technical arts to investigation of the vascular system, for example utilization of pulsed-Doppler ultrasound. Others mimic the ausculatory system by providing an automated approach to the detection and processing of Korotkoff sounds.

The foregoing approaches to the measurement of blood pressure, whether manual or automatic, are well suited to application for a relatively calm, stationary subject. They are not, however, particularly well suited to determination of blood pressure while the subject is engaged in physical exercise, or similar non-stationary activity. Principally, this is so because the physical activity which makes such dynamic measurement desirable, also produces such extensive and unpredictable artifact data that the actual phenomena being measured are swamped and substantially lost. Said otherwise, artifact signals and artifact data produced during the exercise process are so prevalent that the signal to noise ratio becomes less than unity, rendering the data substantially meaningless.

It is appropriate briefly to consider the source and types of artifact data generated during physical exercise, which severely complicate the task of measuring blood pressure during exercise. To do so, one need only consider the mechanics of an individual on a treadmill. Even for the most muscular and physically fit of subjects, and to a much greater extent for less firm and fit subjects, the process of running involves extensive and generally irregular absorption by and passage through the body of shock waves attendant on the rising and falling of feet. Also, as these fluctuations and displacements pass through the body, the subject typically is contorting and displacing the various body parts in many different directions, including forward and backward bending, side to side swaying, head motion, arm pumping, and the like. The net result of all this motion, from the standpoint of the blood pressure cuff, is a complex, unpredictable, and often high amplitude noise signal which has no real bearing on blood pressure. Similarly, during the running process, the very pumping of the arms results in expansion and contraction of the arm in the biceps/ triceps regions (which still appears to be the preferable location for blood pressure monitoring), making it difficult for the automatic blood pressure measurement apparatus even to identify the actual cuff pressure, assuming the ability adequately to draw a signal from the noise and recognize the proper indicia of blood pressure parameters.

Many prior art attempts to measure blood pressure accurately during physical exercise have concentrated on utilization of filtering. That is, one class of prior art systems employs sophisticated band pass filtering of the noisy signal, attempting selectively to eliminate motion artifact signals while passing true data such as Korotkoff sounds. This approach, however, has been uniformly unsuccesful, principally because the motion artifacts involve a frequency spectrum substantially co-extensive with the frequency spectrum of Korotkoff sounds (which themselves are extremely complex).

Another approach to the problem of signal discrimination and noise elimination has been the employment of ECG gating. That is, electrocardiograph electrodes are placed on the subject, allowing for an ongoing monitoring of the "PQRST" cardiac complex. Since Korotkoff sounds result from a rush of arterial blood being pumped through an artery, a knowledge of the moment-to-moment condition of the heart will correspondingly identify but a limited portion of time during which the Korotkoff sound could occur. ECG gating techniques have provided a viable and at times valuable approach to blood pressure measurement for a quiescent or stationary subject, but for a subject during exercise, they provide no substantial relief to the problem of elimination of previously described artifact data.

For an elaborate presentation of the physiology of blood pressure measurement, and the theory and practice of many approaches to such measurement, including ECG gating, reference may be had to the textbook "The Direct and Indirect Measurement of Blood Pressure", by L. A. Geddes, Yearbook Medical Publishers, Inc., Chicago (1970), which also includes a rather extensive bibliography on the subject.

It is a primary object of the present invention to provide apparatus for automatically measuring the blood pressure of a subject during periods of intense physical activity, such as cardiac stress testing on a treadmill, cycle, or the like. It is a related object to provide such automatic measurements with relatively minimal intrusion into the exercise process, allowing the exercise routine to continue uninterrupted, and providing a relative minimum of physical limitation or encumberance.

It is an associated object of the present invention to provide automatic blood pressure signal processing techniques whereby true and valid data relating to blood pressure parameters is drawn or discriminated from noise, such as noise attendant on physical motion during exercise.

Description of the Invention

The present invention is grounded on the proposition that numerous prior art techniques, such as incremental cuff deflation, sonic monitoring of arterial conditions, ECG gating, and digital signal processing techniques, may be combined, improved, and built upon to yield an apparatus which rapidly and accurately measures blood pressure during periods of intense physical exercise, with relatively minimal inconvenience or discomfort to the subject. The apparatus of the present invention is set forth in claim 1.

In particular, the apparatus of the present invention utilizes in part a digital signal processing technique known as non-phase-coherent correlation detection. This technique is set forth in general terms in "Comparison of Phase-Coherent and Non-Phase-Coherent Coded Communications" by J. P. Strong and T. V. Seliga, Goddard Space Flt. Ctr. Publication No. X-711-65-425, Oct. 1965, and generally involves repeated signal sampling at respective times of low and high probability of occurrence of a signal to be sampled, individual processing of the respective sample sets, and a comparison of the results of such processing. Hence, the technique effectively develops separate indices or representations of noise, and signal (if any) plus noise, with the consequent ability to identify the differential index comprising signal.

In accordance with the present invention, the apparatus provides ECG electrodes (e.g. three in number) for placement on the subject in conventional fashion to monitor cardiac function and to produce a signal representative of the PQRST complex. A pressure cuff, having a microphone on its interior surface, is provided for placement on a limb (e.g. the arm) of the subject, with the microphone positioned for sonic monitoring of arterial noises. The cuff is capable of being coupled to a pump through a reservoir of substantial volume, which tends to minimize spurious pressure changes due to volumetric changes in the cuff occasioned by muscle flexing and the like. The cuff can be pumped to a pressure above systolic (e.g. a pressure which substantially impedes blood flow in the artery being monitored), and thence can be deflated in pressure increments. At each such decrement, and for a number of heart beats dependent upon the heart rate of the subject, each cardiac R-wave can be utilized to enable or gate several samplings of the microphone signal, including accumulating a first plurality of signals (e.g. sixteen samples occurring just before and after the R-wave), and a subsequent, second plurality of samples (e.g.

thirty-two samples occurring a predetermined time subsequent to the first plurality). When this sampling has occurred and reoccurred for the predetermined number of beats (i.e. R-waves), the first plurality can be assembled by suitable averaging techniques to a first index, indicative of noise, and the second plurality is likewise processed to yield a second index indicative of signal (if any) plus noise. The cuff pressure level at which the second index exceeds the first by a predetermined amount can be identified as the first level at which Korotokoff sound has occurred, in other words, systolic pressure. Optionally, such detection of systolic pressure can be verified by a repetition of the process at the next decrement, but in any event, once systolic pressure is identified with certainty, the process can be continued in similar fashion for detection of diastolic pressure. The key to detection of diastolic pressure is identification of a pressure level at which the second index achieves a specified decreasing rate of change with respect to the first, or ceases to exceed the first by a predetermined amount. Once diastolic pressure is identified, the cuff can be deflated, and at a subsequent time, the process can be reinstituted.

The apparatus of the present invention permits several operational features which enhance the accuracy and shorten the necessary time of measurement, and promote increased comfort for the subject. For example, depending upon the information at hand, the step decrements in cuff pressure may be substantially varied in amplitude, and, in accordance with specified criteria, many pressure levels intermediate the systolic and diastolic levels may be disposed of completely.

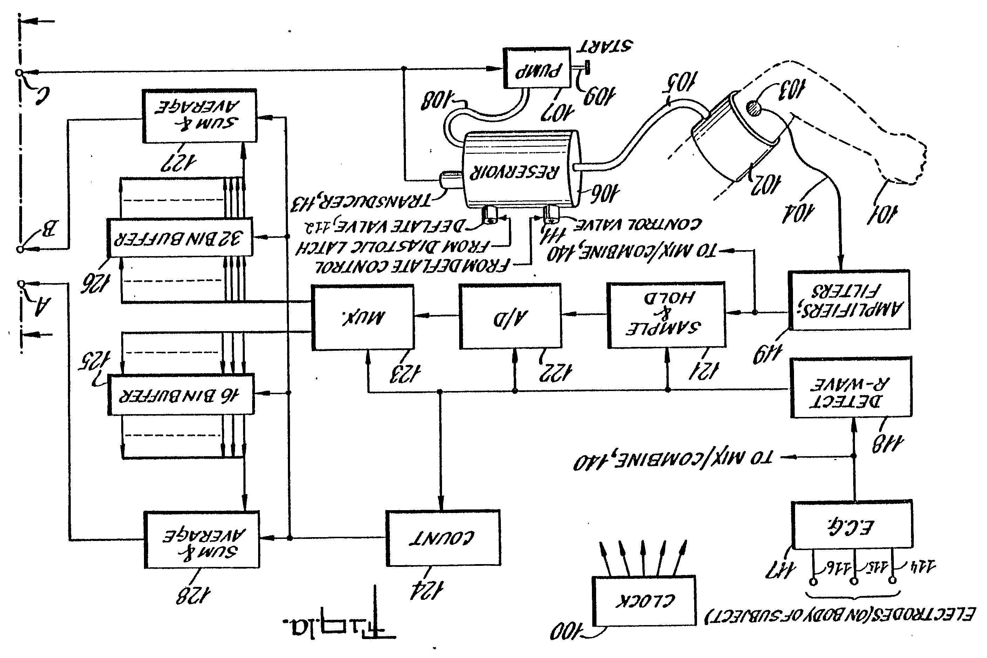

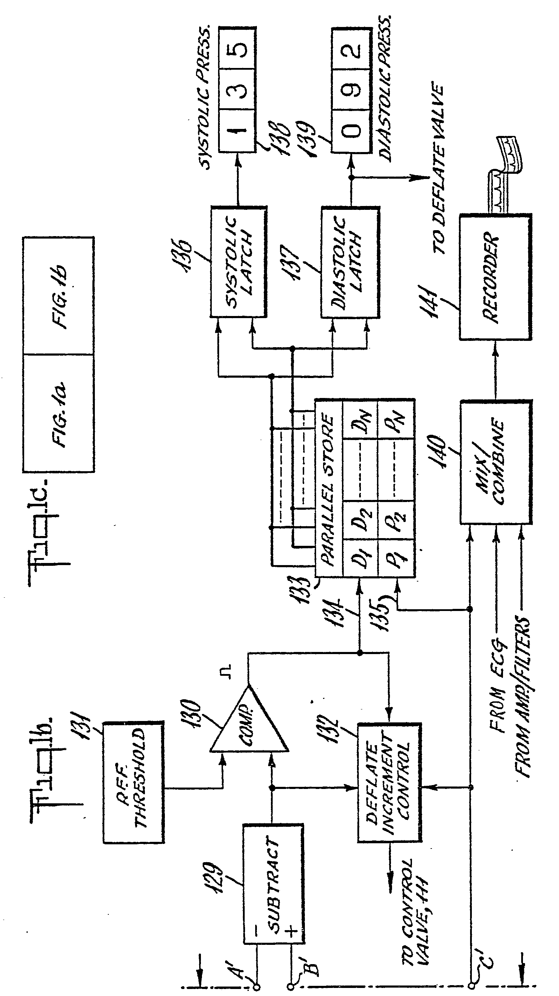

These and other features will be readily apparent upon consideration of the following detailed description, in conjunction with the accompanying drawings, in which Figures 1A and 1B, when joined in the fashion shown in Figure 1C show by way of example only one embodiment of apparatus according to the present invention.

Considering first the mechanical and electromechanical aspects of the embodiment of Figs. 1A and 1B, an inflatable pressure cuff 102 is adapted to be worn on a suitable appendage of the subject, such as the arm 101 shown in phantom. A microphone 103 is mounted on the inner surface of the cuff, whereby the cuff is placed on an arm with the microphone 103 overlying or substantially adjacent a principal artery such as the brachial artery. The microphone is connected by means of wire 104 to conventional signal conditioning apparatus 119, such as amplifiers and filters. The cuff 102 is also connected by means of a hose 105 to a reservoir 106, which in turn is coupled by a hose 108 to a pump 107. The purpose of the reservoir 106 and hose 105, in conjunction with the cuff 102, is to provide a substantial gross volume of air which establishes pressure in the cuff 102, whereby muscle expansion and contraction of the arm 101 has substantially minimal effect upon the fluctuation of cuff pressure. Hence, the hose 105 preferably has a substantial diameter, and the reservoir 106 preferably has a volume substantially in excess of the volume of the cuff 102, whereby changes in cuff volume engender a rather insubstantial change in total volume (volume of cuff 102 plus volume of hose 105 plus volume of reservoir 106) and consequent minimal change in cuff pressure. Further, the cuff 102, hose 105, and reservoir 106 share exchange of air and pressure freely, for practical purposes constituting but a single chamber (i.e. "volumetrically integral").

As shown, the reservoir has attached thereto a control valve 111, a deflate valve 112, and a pressure transducer 113. The control valve 111, when opened as discussed hereinafter, accomplishes the decrementing of cuff pressure by controlled release of air from the reservoir 106. The deflate valve 112, which typically is substantially larger than the control valve 111, accomplishes the final deflation of reservoir 106 and cuff 102 after both systolic and diastolic pressure have been measured. The transducer 113 senses the air pressure in the reservoir 106 (and correspondingly also in the cuff 102), and produces an electrical signal representative of that pressure. The transducer signal, therefore, is the one which, at appropriate times, is identified as representing the systolic and diastolic pressures of the subject.

The pump 107, which is energized by a start switch 109, establishes pressure within the reservoir 106, hose 105, and cuff 102 via connecting hose 108. The pump 107 senses the signal from transducer 113, in order properly to establish an initial pressure in the cuff 102. Upon actuation of the start switch 109, the pump 107 establishes within the cuff 102 a pressure which is in excess of systolic pressure, preferably a pressure which constricts the artery monitored by microphone 103 and prevents blood flow therethrough. Subsequently, upon receipt of control pulses from deflation control unit, the control valve 111 reduces the pressure in the cuff 102 in increments.

In accordance with the knowledge of those of ordinary skill, a clock 100 is shown coupled generally to the apparatus, its purpose being to provide control and regulation of timing for all the apparatus.

An electrocardiograph (ECG) 117 is shown schematically, with three electrodes 114, 115, and 116 suitably located on the body of the subject, in conventional fashion, to produce an output signal representative of cardiac function. Such signal is known as the PQRST complex. The "R" aspect of the signal, known as the "R-wave", is a signal impulse which denotes the depolarizing of the cardiac ventricles. Such pulse is preceded by the Q-wave, denoting the wave of atrial contraction arriving at the ventricles, and is terminated at the time of the S-wave. The so-called S—T interval denotes the electrical recovery time of the ventricles or the time intervals between the end of ventricular depolarization and beginning of repolarization.

In accordance with the present invention, detection of the R-wave at 118 is utilized for purposes of "ECG gating", whereby representations of noise and spurious artifacts are obtained just before and/or just after the occurrence of the R-wave, and representations of signal plus noise and spurious signal are obtained a predetermined time thereafter (e.g. generally during the S—T and/or P—R intervals).

Each detected R-wave is coupled to a counter 124, to a sample and hold circuit 121, to an analog to digital converter 122, and to a multiplexer 123. The microphone signal is sampled at 121 via signal conditioning circuitry 119, and the samples are suitably converted to digital representations at converter 122. The digital representations of samples are coupled to a multiplexer 123, which for a time stores and then selectively distributes digital samples to either of two buffers 125 and 126, at the times, under control of the R-wave detector 118, during which a sample is to be utilized.

In accordance with the present invention, for each R-wave, a first plurality of samples (i.e. 16 in number for the embodiment of Figs. 1A and 1B) is to be accumulated and stored in respective bins of the buffer 125, with the samples having a fixed time relationship to the R-way, preferably just before and just after the R-wave at equal intervals during a one-tenth second period. The embodiment of Figs. 1A and 1B also employs, for each R-wave, accumulation and storage of a second plurality of samples (32 in number in the embodiment of Figs. 1A and 1B) in respective bins of the second buffer 126, which occurs at a time period after occurrence of the R-wave, preferably at equal intervals during a 225 millisecond period commencing upon accumulation and storage of the first plurality of samples.

Preferably, the multiplexer 123 has capacity for temporary storage, of a plurality of samples (e.g. at least 8) whereby, upon detection of an R-wave, those 8 samples just before such detection are available for storage in the first eight bins of the buffer 125. Likewise, under control of the R-wave detector 118, counter 124, and clock 100, the 8 samples immediately after the detected R-wave are so stored in buffer in the next 8 bins of buffer 125, and 32 next subsequent samples are distributed to and stored in respective bins of the buffer 126. In turn, this procedure is to be followed for a number of times (e.g. 4 to 10) at each increment of the cuff 102, depending on the heart rate of the subject.

In operation, then, for each R-wave, and therefore for each heart beat, a first succession of samples is distributed to respective bins of the buffer 125, and a second succession of samples is distributed to the bins of the second buffer 126. For the next detected R-wave, and for a number of heart beats, another series of samples, having the same temporal relationship to the detected R-wave, is likewise stored in the buffers 125 and 126. Accordingly, after plural (e.g. between 4 and 10) reiterations of the R-wave triggered storage in buffers 125 and 126, each bin of the buffer has correspondingly stored therein a plurality (e.g. between 4 and 10) digitally coded samples, each sample in a given bin having substantially an identical temporal relationship with an R-wave. Each set of samples within a bin, therefore, is a representation of signal or noise level, occurring on a recurrent basis at that particular cuff pressure level or increment. As stated, between 4 and 10 R-waves are so sampled, and correspondingly between 4 and 10 samples are so accumulated in each bin, because at a given level of exercise, and for a given time duration (i.e. related to the time duration at which a given pressure level is to be maintained at the cuff 102), each individual subject will sustain a different heart beat rate, and the numbers of separate samples in each bin of the buffer will be roughly in proportion to that heart rate.

Referring again to the drawings, samples from the buffers 125 and 126 are conveyed to respective sum and average circuits, 128 and 127. The sum and average circuit 128 has the function of producing a first index, from the data stored in buffer 125, which is representation of noise. The sum and average circuit 127 has the similar task of producing a second index which is representative of signal plus noise. Upon comparison of these two indices, there results a differential which corresponds to signal free of noise.

It will be understood that, given the extensive accumulation of data in buffers 125 and 126, many different information processing techniques are available for considering all of the data within the buffers and reducing it to a significant single index or collection of indices. A preferred method is as follows. First, the respective quantities stored in each bin of the buffers 125 and 126 are added to one another, yielding 16 separate sums from the buffer 125, and 32 separate sums from the buffer 126. Next, running averages are taken for each buffer 125 and 126. With respect to the data from the buffer 125, the sums from the first four bins are averaged, the sums from the second through fifth are averaged, the sums from the third through sixth are averaged, and the running average is so continued through the buffer 125. A similar running average is accumulated at circuit 127 upon data from the second buffer 126. Thereupon, a key one (e.g. the largest) or combinations of ones (e.g. the average) of the respective running averages accumulated at sum and average circuits 127 and 128 are identified. The index so identified at sum and average circuit 128, representing the aggregate of data taken during or about the occurrence of several successive R-waves, represents noise. The index so identified at the sum and average circuit 127, representing the aggregate of data assembled in the ST and/or P—R intervals, represents signal plus noise. Accordingly, the differential between the two indices represents, for that given cuff pressure level, a representation of signal.

In accordance with the present invention, the respective indices from circuit 127 and 128 are coupled to a subtraction circuit 129, which determines the difference therebetween, coupling that difference both to a comparator 130 and to deflation control unit 132. Considering the former connection first, comparator 130 evaluates the differential index from subtractor 129 with respect to a reference threshold established at 131, thereby to determine whether the differential index from subtraction circuit 129, is sufficiently large to be regarded as a Korotkoff sound. If the differential from subtraction circuit 129 is greater than the threshold level from 131, indicating presence of a Korotkoff sound, a logical 1, or output pulse, is produced by comparator 130; otherwise, a logical 0, or no pulse, is produced. The signal bits for comparator 131 are coupled via line 134 to a parallel storage unit 133 (e.g. a shift register), and for each such bit from the comparator (i.e. $D_1$, $D_2$ ... $D_N$), the cuff pressure then extant, as noted by the transducer 113, is also placed into the associated storage in parallel store 133, via input line 135 (i.e. pressure $P_1$ at which bit $D_1$ was evaluated pressure $P_2$ at which bit $D_2$ was evaluated, and so on).

Accordingly, at any given time, the parallel store 133 maintains in storage a succession of cuff pressure increments ($P_1$, $P_2$ ... $P_N$), and for each, as associated binary bit ($D_1$, $D_2$ ... $D_n$) indicating whether the signal level at the appropriate time was sufficiently greater than the noise level to indicate presence of a Korotkoff sound. It will be appreciated that, in accordance with the needs of the designer, and the capacity of the hardware being utilized, it may be appropriate to store but a few D—P combinations, or many. In a preferred embodiment, three digit-pressure combinations are stored, for use as follows.

It will be apparent that the first logical 1 bit from comparator 130 tentatively indicates first occurrence of Korotkoff sound, and can be utilized to identify the pressure then extant in the cuff as systolic pressure. A preferable, higher security course of action, however, is to identify systolic pressure as the former of the first two successive pressure levels in cuff 102 for which successive logical 1 bits are produced by comparator 130. To this end, in Fig. 1B, a systolic latch 136 is adjusted to register systolic pressure at output display 138 as the first pressure $P_1$ of a $P_1$—$P_2$ combination for which $D_1$ and $D_2$ are both logical ones.

It will likewise be apparent that the first logical zero bit from comparator 130, after a succession of logical 1 bits, tentatively indicates cessation of occurrence of Korotkoff sounds, and such logical zero bit alone can be utilized to identify the pressure then extant in the cuff as diastolic pressure. A preferable, higher security course of action, however, is to identify diastolic pressure as the former of two successive pressure levels in cuff 102, which follow a level having a logical 1 bit from comparator 130, and for which successive logical zero bits are produced by comparator 130. That is, diastolic latch 137 is conditioned to couple to diastolic output display 139 the pressure $P_2$ of a $P_1$—$P_2$—$P_3$ combination for which $D_1$ is a logical 1 and $D_2$ and $D_3$ are both logical zeroes.

An alternative approach to identification of diastolic pressure entails an adaptation of the Fig. 1B apparatus, for direct consideration of the differential indices from subtraction circuit 129. It is known that, just prior to cessation of Korotkoff sounds, there occurs a succession of Korotkoff sounds whose amplitudes rapidly decrease. In accordance with such alternative method for identifying diastolic pressure, then, differences between successive differential indices from subtraction circuit 129 are computed, and they in turn are compared with a stored reference threshold. When such index-to-index differences are greater than the stored threshold, the cessation of Korotkoff sounds is imminent, and diastolic pressure is deemed to occur.

As previously stated, in the embodiment of Figs. 1A and 1B, the differential index from subtraction circuit 129 is coupled not only to comparator 130, but also to deflation increment control 132. The deflation increment control unit performs two functions, first the ongoing regulation of the incremental step size, from cuff pressure level to cuff pressure level, and secondly, providing for larger "one-shot" stepwise decreases subsequent to identification of systolic pressure, but prior to identification of diastolic pressure. In either event, it will be appreciated that these functions performed by deflation increment control are optional, and that the apparatus as heretofore described will provide adequate measurement of systolic and diastolic pressures whether the decrements between cuff pressure levels are regular or irregular.

In the event that the optional deflation increment control is to be provided at 132, its primary rationale is to accelerate the measurement of systolic and diastolic pressures, and to relieve, as quickly as possible, the discomfort of cuff pressure on the arm of the subject. Accordingly, it has been found that decrement sizes from cuff pressure to cuff pressure may be increased roughly in proportion to the amplitude of the Korotkoff sound. Hence, control valve 111 is energized to open for a time roughly proportional to the size of the differential index from subtraction circuit 129. Simple operational amplifier circuitry may be adapted to perform this function, in accordance with the abilities of those of ordinary skill in the art.

It has likewise been found in accordance with empirical criteria, that rather large one-shot decrements may be employed upon identification of systolic pressure, quickly relieving pressure of the cuff, but without substantial danger of overshooting and missing true diastolic pressure. To this end, the deflation increment control 132 may be adapted, upon successful identification of the systolic pressure, to open control valve 111 and thereby to reduce pressure in the cuff 102 to the lower of:

A) 40 mbar (30 mmHg.) pressure less than identified systolic pressure; or

B) 180 mbar (135 mmHg.) pressure.

To this end, bits from comparator 130, as well as the transducer pressure 113, are available to deflation increment control 132. Alternatively, an enabling connection from the systolic latch 136 (not shown in the drawing) may be employed. An additional criterion, if available (i.e. if the systolic-diastolic cycle is being conducted on a repeating basis), is to deflate control valve based either on the two foregoing criteria, or in any event within 33 mbar (25 mmHg.) above the most recent previously measured diastolic pressure.

Upon successful identification of diastolic pressure, an energizing signal is coupled from diastolic latch 137 to the deflation valve 112, thereby expeditiously to relieve pressure in the cuff 102.

As shown in Fig. 1B, a mixer 140 receives, on an ongoing basis, the pressure signal from transducer 113, the ECG signal from 117, and the unsampled microphone signal from signal conditioning circuitry 119. Advantageously, these three analog signals are combined, such as by simple superposition, and the combination is printed such as on strip chart by recorder 141. This aggregate signal may be employed by the clinician as a hard copy of the signals whereby, employing graphical methods, systolic and diastolic pressure may roughly be computed, and checked against the pressures displayed at 138 and 139, if desired.

The above description shows one apparatus according to the invention in hard wired form. It will be appreciated, however, that the apparatus could instead use suitable alternatives.

**Claims**

1. Apparatus, for automatically measuring the blood pressure of a subject, comprising:

(A) a signal processing circuit (118—131) connectable to:

a microphone means (103), on an inner surface of an inflatable pressure cuff (102), for producing a microphone signal representative of noises generated within a limb of the subject to which the cuff (102) is secured;

electrocardiograph electrodes (114—116) for producing ECG signals representative of the cardiac function of the subject;

a pressure sensor (113) for producing a pressure signal representative of the pressure within the inflatable pressure cuff (102; and to

a pressure release valve (111) on the inflatable pressure cuff (102),

the signal processing circuit (118—131) comprising sensing means (118) for sensing the occurrence of an R-wave in the ECG signals,

(B) display control means (133, 136—139) arranged to receive the pressure signal from the pressure sensor (113) and to store and display the value of the pressure within the cuff (102) in dependence on a control signal from the signal processing circuit (118—131), and

(C) deflation control means (132) for controlling·

the pressure relief valve (111) in dependence on the control signal, characterised in that:

the signal processing circuit (118—131) further comprises first and second storage means (125, 126), connected for receiving successive microphone signal samples, controllable in synchronism with the sensing of R-waves by the sensing means (118) so as to store a set of microphone signal samples having a different synchronism with the R-wave in each respective storage means;

processing means (127—129) are connected to the first and second storage means (125, 126), for generating first and second index signals by averaging the microphone signal samples stored in the respective storage means (125, 126) and producing at its output a difference signal determined by the difference between the first and second index signals;

a comparator circuit (130) is connected to said processing means (127—129) for receiving said difference signal and a reference signal and for producing the control signal in dependence on the comparison of the difference signal with the reference signal; and

the display control means (133, 136—139) and the deflation control means (132) are connected to the output of the comparator circuit (130).

2. Apparatus according to claim 1, wherein the signal processing circuit is connected to a recording apparatus (141) so that microphone signal samples, ECG signals and pressure signals are recorded during measurement of the blood pressure.

3. Apparatus according to claim 1 or claim 2, including a reservoir, having a substantially larger volume than that of the cuff, which is substantially volumetrically integral with the cuff, for reducing the significance of spurious volumetric variations in cuff pressure.

4. Apparatus according to any one of claims 1 to 3, wherein:

the signal processing circuit (118—131) is arranged to cause the pressure cuff (102) to be deflated incrementally;

the first storage means (125) is arranged so that at each pressure level it receives a number of first pluralities of microphone signal samples, each first plurality having a first temporal correspondence to each R-wave, and the number being determined by the signal processing circuit (118—131) in dependence on the heart rate derived from the ECG signal;

the second storage means (126) is arranged so that at each pressure level it receives an equivalent number of second pluralities of microphone signal samples, each second plurality occurring a predetermined interval after the corresponding first plurality;

the processing means (127—129) is arranged to combine all said first and second pluralities received at each pressure level to produce said first and second index signals respectively; and

the comparator circuit (130) is arranged to produce the control signal so that the display control (133, 136—139) displays the pressure level at which the second index signal exceeds the first index signal by at least a predetermined factor and the pressure level at which the first and second index signals have a predetermined relationship, these pressure levels being systolic and diastolic pressure respectively.

5. Apparatus according to claim 4, wherein the signal processing means (118—131) is arranged to determine the first successive pressure levels at both of which said second index signal exceeds said first index signal by at least said predetermined factor and to control the display control means (133, 136—139) so that it displays the first of the two successive pressure levels as systolic pressure.

6. Apparatus according to claim 4 or claim 5, wherein the signal processing means (118—131) is arranged so that, once the second index signal has exceeded the first index signal by at least said predetermined factor, the rate at which the cuff pressure is deflated is increased, in accordance with select criteria, until a lower pressure level, which is still in excess of diastolic pressure, is reached.

7. Apparatus according to claim 6, wherein the signal processing means (118—131) is arranged to increase the rate of cuff deflation until the pressure level is less than 180 mbar (133 mm. Hg) pressure or is 40 mbar (30 mm. Hg) pressure less than the determined systolic pressure.

8. Apparatus according to claim 6 or claim 7, wherein the signal processing means (118—131) is arranged to cause the cuff (102) to be reflated and deflated repeatedly *in extenso* and to increase the rate of cuff deflation until the pressure level is in any event at least 33 mbar (25 mm. Hg) above the most recently evaluated diastolic pressure.

9. Apparatus according to any one of claims 4 to 8, and including means (132) arranged to vary the decrement in the pressure level in the cuff (102) in dependence on the amplitude of the second index signal determined by the processing means (127—129) at at least the previous pressure level.

10. Apparatus according to any one of claims 4 to 9, wherein said number of samples is from four to ten.

11. Apparatus according to any one of claims 4 to 10, wherein the comparator circuit (130) is arranged to produce the control signal when said first and second index signals have said predetermined relationship if and only if said second index signal is less than said predetermined factor greater than said first index signal.

12. Apparatus according to any one of claims 4 to 11, wherein the signal processing circuit (118—131) is arranged to automatically rapidly and substantially completely relieve pressure within said cuff (102) after said diastolic pressure has been displayed.

**Patentansprüche**

1. Gerät zur selbsttätigen Messung des Blutdrucks einer Person, mit

(A) einem Signalverarbeitungsschaltkreis (118—131), der verbindbar ist mit:

einer Mikrofoneinrichtung (103) an einer Innenseite einer aufblasbaren Druckmanschette (102) zur Erzeugung eines Mikrofonsignals, welches in einem Glied der Person, dem die Manschette (102) angelegt ist, erzeugte Geräusche darstellt,

elektrokardiographischen Elektroden (114—116) zum Erzeugen von die Herzfunktion der Person darstellenden ECG-Signalen,

einem Druckfühler (113) zum Erzeugen eines den Druck in der aufblasbaren Druckmanschette (102) darstellenden Drucksignals, und mit

einem Druckfreigabeventil (111) an der aufblasbaren Druckmanschette (102),

wobei der Signalverarbeitungsschaltkreis (118—131) eine Erfassungseinrichtung (118) umfaßt, um das Auftreten einer R-Welle in den ECG-Signalen zu erfassen,

(B) einer Anzeigesteuereinrichtung (133, 136—139), die ausgebildet ist, das Drucksignal von dem Druckfühler (113) zu empfangen und den Wert des Druckes in der Manschette (102) in Abhängigkeit von einem Steuersignal von dem Signalverarbeitungsschaltkreis (118—131) zu speichern und anzuzeigen, und

(C) einer Entleerungssteuereinrichtung (132), um das Druckfreigabeventil (111) in Abhängigkeit von dem Steuersignal zu steuern, dadurch gekennzeichnet, daß

der Signalverarbeitungsschaltkreis (118—131) ferner eine erste und eine zweite Speichereinrichtung (125, 126) umfaßt, die zum Empfangen aufeinanderfolgender Mikrofonsignalproben verbunden sind und synchron mit dem Erfassen von R-Wellen mit der Erfassungseinrichtung (118) steuerbar sind, so daß ein Satz von Mikrofonsignalproben unterschiedlichen Synchronismus mit der R-Welle in jeder entsprechenden Speichereinrichtung speicherbar ist,

eine Verarbeitungseinrichtung (127—129) mit der ersten und der zweiten Speichereinrichtung (125, 126) verbunden ist, um erste und zweite Indexsignale zu erzeugen, indem die in den entsprechenden Speichereinrichtungen (125, 126) gespeicherten Mikrofonsignalproben gemittelt werden, und an ihrem Ausgang ein Differenzsignal zu erzeugen, welches durch den Unterschied zwischen dem ersten und dem zweiten Indexsignal bestimmt ist,

ein Vergleicherschaltkreis (130) mit der Verarbeitungseinrichtung (127—129) verbunden ist, um das Differenzsignal und eine Bezugssignal zu erhalten und das Steuersignal in Abhängigkeit von dem Vergleich des Differenzsignals mit dem Bezugssignal zu erzeugen, und

die Anzeigesteuereinrichtung (133, 136—139) und die Entleerungssteuereinrichtung (132) mit dem Ausgang des Vergleicherschaltkreises (130) verbunden sind.

2. Gerät nach Anspruch 1, bei dem der Signalverarbeitungsschaltkreis mit einer Aufzeichnungsvorrichtung (141) verbunden ist, so daß die Mikrofonsignalproben, die ECG-Signale und die Drucksignale während der Blutdruckmessung aufgezeichnet werden.

3. Gerät nach Anspruch 1 oder 2, welches eine Behälter umfaßt, welcher ein wesentlich größeres Volumen als dasjenige der Manschette aufweist und im wesentlichen volumenmäßig mit der Manschette ganzheitlich ist, um die Bedeutung zufälliger, volumetrischer Änderungen beim Manschettendruck zu verringern.

4. Gerät nach einem der Ansprüche 1 bis 3, bei dem:

der Signalverarbeitungsschaltkreis (118—131) ausgebildet ist, ein schrittweises Entleeren der Druckmanschette (102) zu bewirken,

die erste Speichereinrichtung (125) ausgebildet ist, so daß sie bei jedem ersten Druckpegel eine Anzahl erster Mehrheiten von Mikrofonsignalproben erhält, wobei jede erste Mehrheit eine erstes zeitliche Entsprechung mit jeder R-Welle aufweist, und die Anzahl von dem Signalverarbeitungsschaltkreis (118—131) in Abhängigkeit von der von dem ECG-Signal abgeleiteten Herzfrequenz bestimmt wird,

die zweite Speichereinrichtung (126) ausgebildet ist, so daß sie bei jedem Druckpegel eine äquivalente Anzahl zweiter Mehrheiten von Mikrofonsignalproben erhält, wobei jede zweite Mehrheit ein vorbestimmtes Intervall nach der entspechenden ersten Mehrheit auftritt,

die Verarbeitungseinrichtung (127—129) ausgebildet ist, alle ersten und zweiten bei jedem Druckpegel erhaltenen Mehrheiten zu kombinieren, um das erste bzw. zweite Indexsignal zu erzeugen, und

der Vergleicherschaltkreis (130) ausgebildet ist, das Steuersignal zu erzeugen, so daß die Anzeigesteuerung (133, 136—139) den Druckpegel, bei dem das zweite Indexsignal das erste Indexsignal um wenigstens eine vorbestimmten Faktor überschreitet, und den Druckpegel anzeigt, bei dem das erste und das zweite Indexsignal eine vorbestimmte Beziehung aufweisen, wobei diese Druckpegel der systolische bzw. der diastolische Druck sind.

5. Gerät nach Anspruch 4, bei dem die Signalverarbeitungseinrichtung (118—131) ausgebildet ist, die ersten zwei aufeinanderfolgenden Druckpegel zu bestimmen, bei denen jeweils das zweite Indexsignal das erste Indexsignal um wenigstens den vorbestimmten Faktor überschreitet, und die Anzeigesteuereinrichtung (133, 136—139) zu steuern, so daß sie den ersten der zwei aufeinanderfolgenden Druckpegel als systolischen Druck anzeigt.

6. Gerät nach Anspruch 4 oder 5, bei dem die Signalverarbeitungseinrichtung (118—131) ausgebildet ist, so daß dann, wenn das zweite Indexsignal das erste Indexsignal um wenigstens den vorbestimmten Faktor überschritten hat, die Geschwindigkeit, mit der die Druckmanschette entleert wird, in Übereinstimmung mit ausgewählten Kriterien erhöht wird, bis ein niedererer Druckpegel erhalten wird, der noch über dem diastolischen Druck liegt.

7. Gerät nach Anspruch 6, bei dem die Signal-

verarbeitungseinrichtung (118—131) ausgebildet ist, die Geschwindigkeit der Manschettenentleerung zu erhöhen, bis der Druckpegel kleiner als 180 mbar (135 mmHg) oder 40 mbar (30 mmHg) kleiner als der festgestellte systolische Druck ist.

8. Gerät nach Anspruch 6 oder 7, bei dem die Signalverarbeitungseinrichtung (118—131) ausgebildet ist, ein wiederholtes Aufblasen und Entleeren in extenso zu bewirken und die Manschettenentleerungsgeschwindigkeit zu erhöhen, bis der Druckpegel auf jeden Fall wenigstens 33 mbar (25 mmHg) über dem als letzten berechneten diastolischen Druck liegt.

9. Gerät nach einem der Ansprüche 4 bis 8, welches Mittel (132) aufweist, die ausgebildet sind, die Abnahme des Druckpegels in der Manschette (102) in Abhängigkeit von der Amplitude des zweiten Indexsignals zu verändern, welches von der Verarbietungseinrichtung (127—129) bei wenigstens dem vorhergehenden Druckpegel bestimmt worden ist.

10. Gerät nach einem der Ansprüche 4 bis 9, bei dem die Anzahl der Proben vier bis zehn beträgt.

11. Gerät nach einem der Ansprüche 4 bis 10, bei dem der Vergleicherschaltkreis (130) ausgebildet ist, das Steuersignal, wenn das erste und das zweite Indexsignal die vorbestimmte Beziehung aufweisen, dann und nur dann zu erzeugen, wenn das zweite Indexsignal kleiner als der vorbestimmte Faktor größer als das erste Indexsignal ist.

12. Gerät nach einem der Ansprüche 4 bis 11, bei dem der Signalverarbeitungsschaltkreis (118—131) ausgebildet ist, automatisch, schnell und im wesentlichen vollständig Druck in der Manschette (120) zu entfernen, nachdem der diastolische Druck angezeigt worden ist.

## Revendications

1. Appareil pour mesurer automatiquement la pression sanguine d'un sujet comprenant:

A) un circuit (118—131) de traitement de signaux susceptible d'être connecté:

à des moyens microphoniques (103) montés sur une surface intérieure d'un brassard à tension gonflable (102) pour produire un signal microphonique représentatif des bruits engendrés dans un membre du sujet auquel le brassard (102) est attaché;

à des électrodes (114—116) d'électrocardiographe pour produire des signaux ECG représentatifs de la fonction cardiaque du sujet;

à un détecteur (113) de pression pour produire un signal de pression représentatif de la pression qui règne dans le brassard à tension gonflable (112); et

à un robinet de décompression (111) monté sur le brassard à tension gonflable (102);

le circuit (118—131) de traitement de signaux comprenant des moyens détecteurs (118) pour détecter l'apparition d'une onde R dans les signaux ECG;

B) des moyens (133, 136—139) de commande d'affichage agencés de manière à recevoir le signal de pression du détecteur (113) de pression et à mettre en mémoire et afficher la valeur de la pression qui régne à l'intérieur du brassard (102) en fonction d'un signal de commande émis par le circuit (118—131) de traitement de signaux; et

C) des moyens (132) de commande de dégonflage pour commander le robinet de décompression (111) en fonction du signal de commande, caractérisé en ce que:

le circuit (118—131) de traitement de signaux comprend, en outre, des premiers et seconds moyens de mémoire (125, 126), connectés de manière à recevoir des échantillons successifs du signal microphonique, susceptibles d'être commandés en synchronisme avec la détection des ondes R par les moyens détecteurs (118) de façon à mettre en mémoire un ensemble d'échantillons du signal microphonique ayant un synchronisme différent avec l'onde R dans chacun des moyens de mémoire respectifs;

des moyens de traitement (127—129) sont connectés aux premiers et seconds moyens de mémoire (125, 126), pour engendrer des premier et second signaux indicateurs en calculant la moyenne des échantillons du signal microphonique mis en mémoire dans les moyens de mémoire respectifs (125, 126) et en produisant à leur sortie un signal de différence déterminé par la différence entre les premier et second signaux indicateurs;

un circuit comparateur (130) est connecté auxdits moyens de traitement (127—129) pour recevoir ledit signal de différence et un signal de référence et pour produire le signal de commande en fonction de la comparaison du signal de différence avec le signal de référence; et

les moyens (133, 136—139) de commande d'affichage et les moyens (132) de commande de dégonflage sont connectés à la sortie du circuit comparateur (130).

2. Appareil selon la revendication 1, dans lequel le circuit de traitement de signaux est connecté à un appareil d'enregistrement (141) de sorte que les échantillons du signal microphonique, les signaux ECG et les signaux de pression sont enregistrés pendant la mesure de la pression sanguine.

3. Appareil selon la revendication 1 ou la revendication 2 comprenant un réservoir ayant un volume nettement plus grand que celui du brassard, et qui est relié à celui-ci pour former sensiblement une seule chambre, pour réduire l'influence de variations volumétriques parasites sur la pression du brassard.

4. Appareil selon l'une quelconque des revendications 1 à 3, dans lequel:

le circuit (118—131) de traitement de signaux est agencé de manière à provoquer un dégonflage par incréments du brassard à tension (102);

les premiers moyens de mémoire (125) sont agencés de telle sorte qu'à chaque niveau de pression, ils reçoivent un certain nombre de premières séries d'échantillons du signal microphonique, chaque première série ayant une première correspondance temporelle avec chaque onde R et le nombre étant déterminé par le circuit (118—131) de traitement de signaux en fonction de la fréquence cardiaque calculée à partir du signal ECG;

les seconds moyens de mémoire (126) sont agencés de telle sorte qu'à chaque niveau de pression, ils reçoivent un même nombre de secondes séries d'échantillons du signal microphonique, chaque seconde série se produisant un intervalle prédéterminé après la première série correspondante;

les moyens de traitement (127—129) sont agencés de manière à combiner la totalité desdites première et secondes séries reçues à chaque niveau de pression pour produire ledit premier et, respectivement, ledit second signal indicateur; et

le circuit comparateur (130) est agencé de manière à produire le signal de commande de façon que la commande d'affichage (133, 136—139) affiche le niveau de pression auquel le second signal indicateur dépasse le premier signal indicateur d'au moins un facteur prédéterminé et le niveau de pression auquel les premier et second signaux indicateurs sont entre eux dans une relation prédéterminée, ces niveaux de pression étant respectivement la pression systolique et la pression diastolique.

5. Appareil selon la revendication 4, dans lequel les moyens (118—131) de traitement de signaux sont agencés de manière à déterminer les deux premiers niveaux de pression successifs auxquels ledit second signal indicateur dépasse ledit premier signal indicateur d'au moins ledit facteur prédéterminé; et à commander les moyens (133, 136—139) de commande d'affichage de façon qu'ils affichent le premier des deux niveaux de pression successifs comme étant la pression systolique.

6. Appareil selon la revendication 4 ou la revendication 5, dans lequel les moyens (118—131) de traitement de signaux sont agencés de manière que, lorsque le second signal indicateur a dépassé le premier signal indicatuer d'au moins ledit facteur prédéterminé, le taux auquel la pression du brassard est diminuée est accru, en fonction de critères choisis, jusqu'à ce qu'un plus bas niveau de pression qui est encore supérieur à la pression diastolique soit atteint.

7. Appareil selon la revendication 6, dans lequel les moyens (118—131) de traitement de signaux sont agencés de manière à accroître le taux de dégonflage du brassard jusqu'à ce que le niveau de pression soit inférieur à une pression de 180 mbars (135 mm Hg) ou inférieur de 40 mbars (30 mm Hg) à la pression systolique déterminée.

8. Appareil selon la revendication 6 ou la revendication 7, dans lequel les moyens (118—131) de traitement de signaux sont agencés pour provoquer le gonflage et le dégonflage du brassard (102) de manière répétée, in extenso, et à accroître le taux de dègonflage du brassard jusqu'à ce que le niveau de pression soit dans tous les cas supérieur d'au moins 33 mbars (25 mm Ha) à la pression diastolique qui a été évaluée le plus récemment.

9. Appareil selon l'une quelconque des revendications 4 à 8 et comprenant des moyens (132) agencés pour faire varier le décrément du niveau de pression du brassard (102) en fonction de l'amplitude du second signal indicateur déterminé par les moyens de traitement (127—129) au moins au niveau de pression précédent.

10. Appareil selon l'une quelconque des revendications 4 à 9, dans lequel ledit nombre d'échantillons est compris entre quatre et dix.

11. Appareil selon l'une quelconque des revendications 4 à 10, dans lequel le circuit comparateur (130) est agencé pour produire le signal de commande lorsque lesdits premier et second signaux indicateurs sont dans ladite relation prédéterminée si et seulement si ledit second signal indicateur moins la valeur du facteur prédéterminé est supérieur audit premier signal indicateur.

12. Appareil selon l'une quelconque des revendications 4 à 11, dans lequel le circuit (118—131) de traitement de signaux est agencé de façon à relâcher rapidement automatiquement et pratiquement complètement la pression régnant dans ledit brassard (102) après que ladite pression diastolique a été affichée.

Fig. 1a.

ELECTRODES (ON BODY OF SUBJECT)

CLOCK — 100

114 115 116 117

E.C.G.

COUNT — 124

SUM & AVERAGE — 128

TO MIX/COMBINE, 140

118

DETECT R-WAVE

16 BIN BUFFER — 125

119

121 SAMPLE & HOLD

122 A/D

123 MUX.

A

AMPLIFIERS; FILTERS

TO MIX/COMBINE, 140

FROM DEFLATE CONTROL
FROM DIASTOLIC LATCH
DEFLATE VALVE, 112
TRANSDUCER, 113

32 BIN BUFFER — 126

B

CONTROL VALVE, 111

101 104 102

106 — RESERVOIR

SUM & AVERAGE — 127

103 105

109 107

PUMP — 108

START

C

0 029 349

Fig.1b.

Fig.1c.

| FIG. 1a | FIG. 1b |
|---------|---------|

0 029 349